# EUROPEAN PATENT APPLICATION

(11) **EP 2 703 387 A1**
(43) Date of publication of application: **05.03.2014**
(21) Application number: 12181916.3
(22) Date of filing: 27.08.2012
(51) Int. Cl.: C07D 213/38, C07D 413/04, C07D 417/04, C07D 295/096, A61K 31/495, A61K 31/496, A61P 25/28

(54) **Cyclohexyl-substituted piperazine compounds**

(71) Applicant: Biofordrug S.R.L., 70125 Bari (BA) (IT)
(72) Inventor: Colabufo, Nicola Antonio, 70125 Bari BA (IT); Leopoldo, Marcello, 70125 Bari BA (IT); Perrone, Roberto, 70125 Bari BA (IT); Berardi, Francesco, 70125 Bari BA (IT)
(74) Representative: Germinario, Claudio

(57) **Abstract**

The present invention relates to cyclohexyl-substituted piperazine, piperidine and cyclohexane compounds, composition comprising the same and process for their synthesis. The invention also relates to said compounds for use as medicament, in particular for the treatment and/or prevention of early stage of Alzheimer's disease.

## Description

### TECHNICAL FIELD

The present invention relates to a new class of compounds, composition comprising the same and process for their synthesis. The invention also relates to said compounds for use as medicament, in particular for the treatment and/or prevention of early stage of Alzheimer's disease.

### BACKGROUND OF THE INVENTION

Alzheimer's disease (AD) is a progressive neurodegenerative disorder characterized by neuron loss, aggregation of beta-amyloid peptide (Aβ) into plaques, and, intracellular aggregates of phosphorylated tau protein also named neurofibrillary tangles (NFTs). Aβ plaques and the NFTs are the two neuropathological hallmarks of AD mostly studied. Since their accumulation is present when the pathology is in a late phase, their detection can be carried out only after the onset of said disease. Indeed, to date, the anti-AD agents used in therapy are useful to treat the symptoms but not to stop the progression of the pathology.

In this scenario, a useful treatment of AD could be possible by the targeting some biomarkers involved in the first phase of the disorder such as the P-glycoprotein (P-gp) and Toll-like Receptors 4 (TLR4). P-gp is an ATPase protein, belonging to the ATP-Binding Cassette (ABC) transporters family, that use the energy of ATP hydrolysis to efflux drugs and xenobiotics out of cells. P-gp is localized at the apical level of biological barriers in Central Nervous System (CNS) such as blood cerebrospinal fluid barrier (BCSF) and blood-brain barrier (BBB) and in periphery organs involved in metabolism and elimination (pancreas, gut, liver, spleen, kidney). It has been demonstrated that Aβ accumulation in brain parenchyma is a consequence of a decreased activity and or expression of P-gp: Indeed, Aβ is a P-gp substrate and P-gp is able to efflux Aβ out of cells preventing its accumulation in the plaques.

In the first phase of AD progression, the recruitment and activation of microglia is a consequence of Aβ accumulation in brain. Activated microglia is a double-edged sword since protect neurons from toxic substances by degrading them but in the meantime it releases proinflammatory cytokines, chemokines, and reactive oxygen and nitrogen species that are harmful to neurons.

Aggregated Aβ activate microglia by cell surface receptor complexes that involve several toll-like receptors (TLRs), in particular TLR4.

A recent study reported by Song et al. (Song et al. Journal of Neuroinflammation 2011, 8:92) demonstrated that TRL4 plays an important role in neuroprotection and therefore, its impairment contributes to Alzheimer's progression. In fact, the authors observed that mutations of TRL4 reduce microglia activation, increase Aβ deposits and exacerbates cognitive deficits in a mouse model of Alzheimer's disease. As a consequence, the activation of microglia by TRLR4 protect neurons form toxic Aβ since, as the authors concluded, TRL4-actived microglia clears Aβ oligomers and deposits present in the (CNS).

Therefore, as indicated by several data of the literature it is possible to state that the first phase of the Alzheimer's disease is characterized by a decreased activity/expression of P-gp and an activation of microglia TLR4-mediated, probably due to decrease of TRL4 expression.

Furthermore, several studies have demonstrated a link between TLR4 and P-gp protein (Heemskerk, S. et al. Regulation of P-glycoprotein in renal proximal tubule epithelial cells by LPS and TNF-alpha. J. Biomed. Biotechnol. 2010; 2010:525180. al. 2010*.* Mishra, J.et al.. Lipopolysaccharide increases cell surface P-glycoprotein that exhibits diminished activity in intestinal epithelial cells. Drug Metab Dispos. 2008, 36, 2145-2149). Indeed, LPS, a TLR4 agonist, induces in intestinal epithelial cells a mobilization of P-gp to the plasma membrane (Mishra et al. 2008) thus, increasing the number of efflux sites for drugs and xenobiotics out of cells.

Scope of the present invention is to fill the gaps left opened in the prior art.

### SUMMARY OF THE INVENTION

The present invention is based on the discovery of a novel family of compounds able to positively modulate both P-gp (P-glycoprotein) and/orTRL4 (Toll Like Receptor 4) expression. In particular, the invention herein disclosed is based on the known relationship existing between P-gp and TRL4 proteins and the importance that such molecules have in the onset of neurodegenerative diseases wherein accumulation and/or deposits of beta-amyloid peptide (Aβ) are observed.

Starting from these two background information, the inventors have developed a new class of compounds that strongly induces the expression of both P-gp and (?) TRL4 proteins, with also a moderate increase in Pg-p activity, and thus, that are capable of decreasing Aβ accumulation into Central Nervous System (CNS).

Said class of compounds, depicted in formula I below, acts as P-gp ligands and as P-gp expression and/or activity enhancer, are able to re-activate the P-gp efflux pump that is responsible of efflux of Aβ out of CNS. Accordingly, these compounds are capable of reducing Aβ deposits that, as known, are toxic for neurons. Furthermore, at the same time these compounds increase TLR4 expression that by contributing to microglia activation, has a protective role towards neurons, as already reported above.

As a consequence of these effects, and as demonstrated in the examples below, the class of compounds developed by the inventors may be useful for treating and/or preventing diseases characterized by a decrease of P-gp and/or TRL4 such as some neurodegenerative diseases. Moreover, experiments carried out by the inventors demonstrate that this new class of compounds displays a decreased effect on P-gp expression after silencing TLR4 and a decreased effect on TLR4 when P-gp is silenced. These findings confirmed the reported link between the expression of the two proteins and suggest that these compounds should be used as a new tool in therapies wherein both proteins have to be targeted.

The pharmacological activity of these compounds is highly advantageous in terms of new therapeutic approach towards neurodegenerative diseases involving Aβ accumulation and/or formation of Aβ deposits/plaques basically due to decrease in P-gp expression-activity and/or TRL4 expression. Indeed, both P-gp and TRL4 protein play an important role in cleaning CNS areas from beta amyloid plaques, as above indicated.

Since in Alzheimer's disease the impairment of both P-gp and TRL4 occurs, in particular in the early stage of the pathology's onset, thus well before the detection of Aβ deposits/plaques be possible, the invention advantageously describes for the first time a new class of compounds useful for the early treatment of Alzheimer's disease (AD).

Hence, object of the present invention is a family of compounds having general formula (I) as depicted in claim 1.

A second object of the invention is a family of compounds having general formula (I) as depicted in claim 1 for use as a medicament, in particular for use in the treatment of early stage of diseases characterized by decrease in P-gp expression and/or TLR4 expression.

A third object of the present invention is a pharmaceutical composition comprising a compound of formula I and at least one pharmaceutical acceptable excipient. In particular a pharmaceutical composition for use in the treatment and/or prevention of neurodegenerative diseases, for instance diseases characterized by a decrease in P-gp and/or TLR4 expression, accompanied or not by Aβ accumulation and/or , formation of deposits/plaques.

A further object of the present description is method for synthesizing the compounds of formula I.

A still further object of the present description is a compound of formula VI and its use for the synthesis of the compound of formula I.

Other objects will be made evident in the light of the following detailed description.

### FIGURE

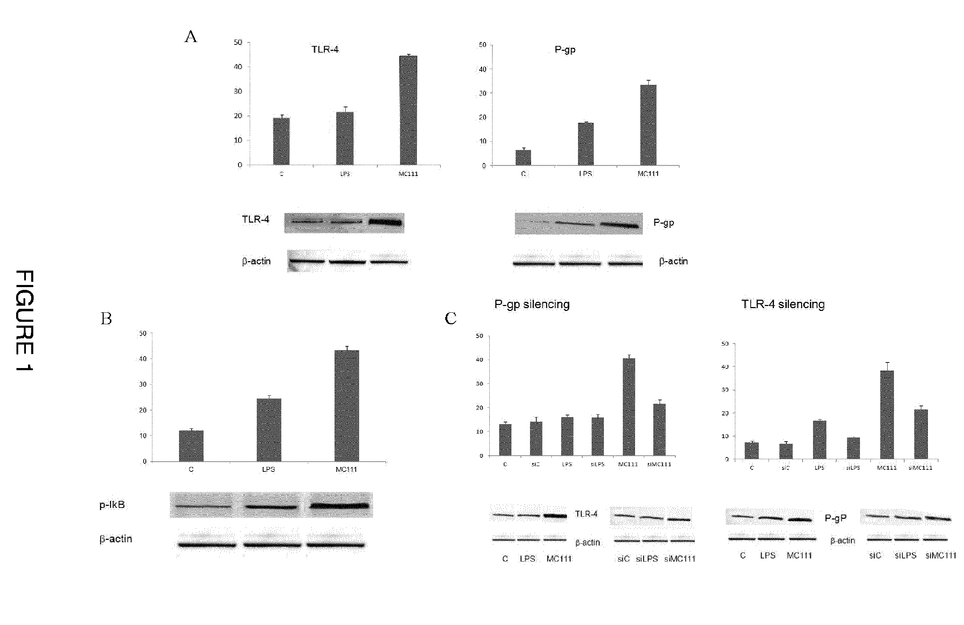

**Figure 1A****-C:** Densitometric analysis of western blotting bands obtained from SW480 cells: Panel A: TLR-4 (left) and P-gp (right) expression in SW480 cells (Control (c): untreated cells; LPS: cells stimulated with 1 µg/ml LPS; cells stimulated with 250 µM of compound of formula II also indicated in this description as MC111. Panel B: Activation of Nf-kB performed by analysis of active phosphorylated form (p-IkB). Panel C: Modulation of TLR-4 expression after P-gp silencing (left) and P-gp expression after TLR-4 silencing (right), respectively (siC: P-gp or TLR-4 silenced untreated cells; siLPS: P-gp or TLR-4 silenced cells stimulated with 1 µg/ml LPS; P-gp or TLR-4 silenced cells stimulated with 250□ µM MC111).

### DETAILED DESCRIPTION OF THE INVENTION

### Class of compounds

The invention relates to a family of novel chemical compounds having the following general formula I 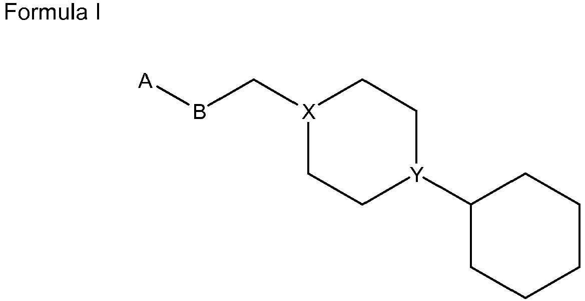 wherein:
- **A** is selected from: 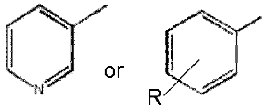
- **B** is selected from: 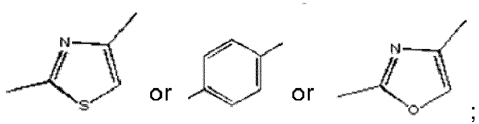 and
   wherein **R** is: H, OH, OCH₃, Cl, Br, F; or NO₂ ; and
- **X** and **Y** are selected from CH or N

In an embodiment of the invention **A** or **B** are phenyl and/or **X** or **Y** are N.

In another embodiment of the invention both A and B are phenyl, or both X and Y are N.

In a preferred embodiment both A and B are phenyl and both X and Y are N.

In one embodiment of the invention, the compound according to formula I is 4'-(4-cyclohexyl-piperazin-1-yl-methyl)-biphenyl-4-R or 1-[2-(3-R)oxazol-4-yl-methyl]-4-cyclohexylpiperazine respectively shown in the following formula II and III. 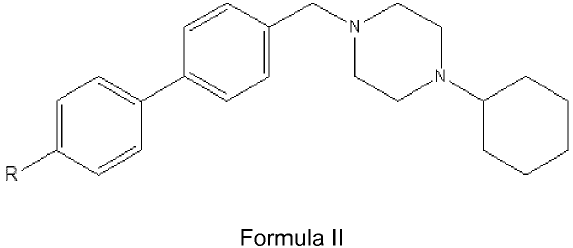 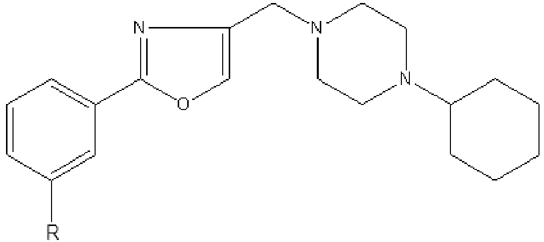 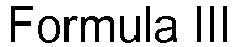 wherein R is: H, OH, OCH₃, Cl, Br, F; or NO₂.

In a preferred embodiment, in the above formula II R is OH, indicated in the present description as MC111.

In another preferred embodiment, in the above formula III R is Cl, indicated in the present description as IG27

Preferably, the compounds of general formula I may be selected from:
4'-(4-cyclohexyl-piperazin-1-yl-methyl)-biphenyl-2-R;
4'-(4-cyclohexyl-piperazin-1-yl-methyl)-biphenyl-3-R;
4'-(4-cyclohexyl-piperazin-1-yl-methyl)-phenyl-pyridine;
1-[2-(3-R-phenyl)oxazol-4-yl-methyl]-4-cyclohexylpiperazine;
1-[2-(2-R-phenyl)oxazol-4-yl-methyl]-4-cyclohexylpiperazine;
1-[2-(4-R-phenyl)oxazol-4-yl-methyl]-4-cyclohexylpiperazine;
1-[2-piridin-oxazol-4-yl-methyl]-4-cyclohexylpiperazine;
1-[2-(2-R-phenyl)tiazol-4-yl-methyl]-4-cyclohexylpiperazine;
1-[2-(3-R-phenyl)tiazol-4-yl-methyl]-4-cyclohexylpiperazine;
1-[2-(4-R-phenyl)tiazol-4-yl-methyl]-4-cyclohexylpiperazine;
1-[2-piridin-tiazol-4-yl-methyl]-4-cyclohexylpiperazine;
wherein in each compound R is selected from H, OH, OCH₃, Cl, Br, F; or NO₂.

### Synthesis method.

The compounds of general formula I have been synthesized by reducing the compound of formula VI to obtain the compound of formula I, according to the following scheme (Scheme I).

Furthermore, the method may comprise a step of reacting compound of formula IV with compound of formula V to generate the intermediate compound VI according to the following Scheme I:

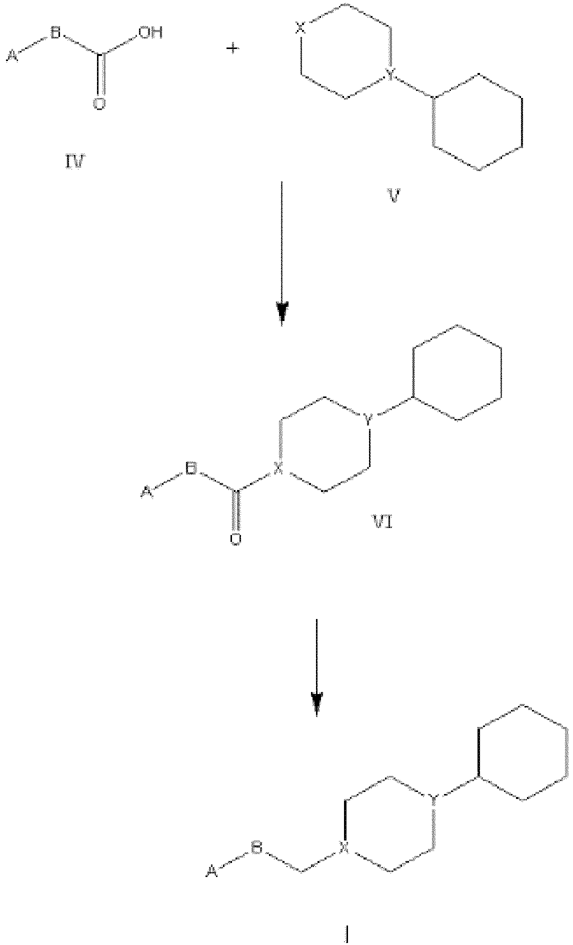

In one embodiment of the present invention, the method further comprised a step for purifying the compound of formula I.

As reported in the scheme above the compound of formula VI is an intermediate and therefore a key compound in the synthesis of compounds of general formula I. Accordingly, another object of the present description is said compound of formula VI. Furthermore, it is also an object of the description the use of said compound of formula VI for the synthesis of compounds described by general formula I.

### Compounds for use as a medicament

The compounds selected from the general formula I above indicated may be use as a medicament since they can act towards both p-glycoprotein (Pg-p) and/or Toll like receptor 4 expression (TRL4), namely two proteins actively involved in some diseases.

In particular, as shown in the experimental section below, the compounds of general formula I are able to highly increase the expression of both Pg-p and TRL4 in an *in vitro* model.

More in details, the compounds of formula I are used in the treatment and/or prevention of any diseases characterized by a decrease in Pg-p and/or TRL4 expression. Examples of diseases wherein a decrease in Pg-p and/or TRL4 expression may be observed are neurodegenerative diseases such as neuroinflammation focal cerebral ischemia and Nasu-Hakola disease.

Since the decreased expression of these two proteins has been linked, in some cases, to an accumulation of beta-amyloid (Aβ) peptide in the CNS (Central Nervous System) and thus, to the formation of Aβ plaques, advantageously the compounds herein described may be use in the treatment and/or prevention of any diseases characterized by an accumulation of Aβ peptide.

Preferred disease to be treated and/or prevented by the compounds object of the present invention, is Alzheimer's disease (AD).

As in AD patients the impairment of P-gp and TRL4 expression has already observed in the early stage of the pathology when Aβ deposits have not so evident yet, advantageously the new class of compounds of general formula may be used for the early treatment of Alzheimer's disease.

### Pharmaceutical compositions

A further object of the invention is a pharmaceutical composition comprising the compounds selected from the above-indicated family and at least one pharmacologically acceptable excipient. Preferably these compositions are in unit dosage forms such as tablets, pills, capsules, powders, granules, sterile parenteral solutions or suspensions, metered aerosol or liquid sprays, drops, ampoules, auto-injector devices or suppositories; for oral, parenteral, intranasal, sublingual or rectal administration, or for administration by inhalation or insufflation. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, acetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin. Thus, based on the above, a variety of pharmaceutically acceptable doses are provided.

Compositions comprising compounds of Formula I and a pharmaceutical acceptable carrier may advantageously be used in the treatment and/or prevention of any diseases characterized by a decrease in p-glycoprotein expression and/or prevention of diseases characterized by a decrease in Toll like receptor 4 expression. Furthermore, the pharmaceutical composition herein described may be in the treatment and/or prevention of any diseases characterized by an accumulation of beta-amyloid peptide. Preferably, said composition may be used for treating and/or preventing Alzheimer's disease. Advantageously, the treatment is an early stage treatment of said diseases, in particular of AD disease.

Unless otherwise specified, when referring, to the compounds of formula I per se, to any pharmaceutical composition thereof, the present invention includes all of the salts, hydrates, solvates, complexes, and prodrugs of the compounds of the invention. Prodrugs are any covalently bonded compounds, which releases the active parent pharmaceutical according to formula I.

### Method for treatment

The method for treating and/or preventing a neurodegenerative disease by modulating P-glycoprotein and/or TLR4 expression, comprises administering to a patient an effective amount of a compound of the families described above. In particular, the method is directed to treatment and/or prevention of any diseases wherein an accumulation of Aβ peptide is observed e.g. Alzheimer's disease.

In one embodiment of the invention, the method involves treatment and/or prevention of early stage of such diseases, preferably early stage of AD.

In the method of treatment the effective amount administered and frequency of administration of the compounds of the present invention will depend on the particular condition to be treated, the severity of the condition, the stage of the disease, age, weight and the overall physical condition of the particular patient as well as on other medicaments the patient is taking, as it is well known to the experts in the field. For illustrative purpose only, a suitable dosage may be comprised in the range from about 10µg/day to about 10mg/day.

### EXAMPLES

### Example 1: Synthesis of compounds of formula II (4'-(4-Cyclohexyl-piperazin-1-yl-methyl)-biphenyl-4-ol).

Compound of formula II above reported has been synthesized according to the following scheme 1.

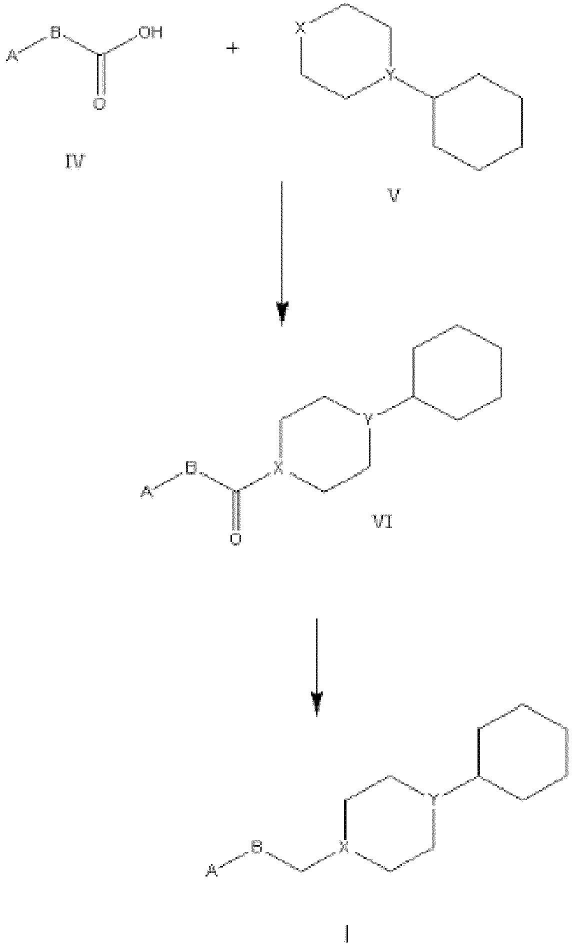

In particular, a solution of carboxylic acid 1 (1 mol) commercially available and N,N-carbonyldiimidazole (1.1 mol) in dry THF was stirred at room temperature overnight. A solution of 1-cyclohexylpiperazine 2 (1 mmol) in THF (tetrahydrofuran) was added at the mixture and the reaction was stirred at room temperature for 6 hours. The solvent was evaporated and the residue was dissolved in H₂O (50 mL). The aqueous phase was extracted with AcOEt (3 x 50 mL). The combined organic solution was dried over Na₂SO₄ and evaporated. The amide so obtained was purified by column chromatography on silica gel (CHC13/MeOH 19:1) obtaining a yellow solid (30%). A solution of the amide (1 mmol) in dry THF (10 mL) was added drop by drop to a suspension of LiAlH4 (2 mmol) in dry THF (5 mL). The reaction was refluxed for 3 hours. After cooling at room temperature water (10mL) was added. The aqueous phase was extracted with AcOEt (3 x 50 mL) and the combined solution was dried over Na2SO4 and evaporated. The compound of formula III ((4-Cyclohexyl-piperazin-1-yl)-(4'-hydroxy-biphenyl-4-yl)-methanone) was purified by column chromatography on silica gel (CHCl₃/MeOH 19:1) obtaining a white solid (55%).

### (4-Cyclohexyl-piperazin-1-yl)-(4'-hydroxy-biphenyl-4-yl)-methanone (compound of formula VI)

ESI+/MS m/z: 365 [M+H]+. ESI+/MS/MS m/z: 240 (13), 197 (100). 1 H-NMR (CDCl3) δ: 1.07-1.31 (m, 5HEq cyclohexyl), 1.62- 1.89 (m, 5H, Hax cyclohexyl), 2.29-2-39 (m, 1 H, NCH cyclohexyl), 2.62-2.65 (m, 4H, CH2NCH2 piperazine), 3.50-3.62 (m, 4H, CH2NCOCH2 piperazine), 3.86 (s, br, 1H, OH), 7.10-7.89 (m, 8H, HAryl). Tdec 170-174°C.

### 4'-(4-Cyclohexyl-piperazin-1-yl-methyl)-biphenyl-4-ol (compound of formula II)

ESI+/MS m/z: 351 [M+H]+. ESI+/MS/MS m/z: 183 (100), 169 (43). 1H-NMR (CDCl3) δ: 1.10-1.30 (m, 5HEq cyclohexyl), 1.43 1.81 (m, 5H, Hax cyclohexyl), 2.03-2.17 (m, 5H, OH cyclohexyl, CH2NCH2 piperazine), 2.57-2.87 (m, 5H, CH2NCOCH2 piperazine, OH), 3.68 (s, 2H, NCH2) 6.87-7.60 (m, 8H, HAryl). Hydrochloride salt recrystallized from MeOH/Et2O. m.p. 290-293°C. Anal. [C23H30N2O2×(HCl)1.5] C, H, N.

### Biological assays

### Cell cultures

The human colon adenocarcinoma cell line SW480 (ICLC HTL99017-Interlab Cell Line Collection) was cultured on Leiboviz-15 medium supplemented with 10% fetal bovine serum, 100 U/ml penicillin, 100 mg/ml streptomycin and L-glutamine (2mM; Life Technologies-Invitrogen). Cell line SW480has been chosen as model in our in vitro experiment since, as well known to a technician, their cells express high level of TRL4 protein and they represent a validated cell model of blood-brain barrier cells.

Cultures were maintained at 37°C in a humidified atmosphere containing 5% CO2 and expanded in tissue culture flasks (75 cm²; BD Biosciences), changing the medium daily. The cells were seeded in six-well cell culture plates at 5x105 cells/well and cultured to reach 80% confluency.

### Cell viability assay

The viability of the cells was assessed by the 3,4,5-dimethylthiazol-2-yl-2-5-diphenyltetrazolium bromide (MTT) assay, which is based on the reduction of MTT by the mitochondrial dehydrogenase of intact cells to a purple formazan product. Cells (2·5 x 10⁴) were seeded in a ninety-six-well plate (BD Biosciences). After the treatment previously described, culture media were carefully removed by aspiration.

Following this, 100 ml of 0·5 mg/ml MTT in cell culture medium were added to each well and incubated for 2 h.

Then, 100 ml of 10% SDS, 0·01 M-HCl solution were added to each well to dissolve the formazan crystals formed. The plates were covered with aluminium foil and kept in an incubator for 12 h for dissolution of the formed formazan crystals.

The amount of formazan was determined by measuring the absorbance at 560nm using a microplate reader.

### Cell treatment

Cell cultures were submitted to following treatments: MC111 at concentration ranging of 250 mM, after preliminary experiments performed in order to establish optimal dose for cell treatment.

Unstimulated cells were used as controls. Cell cultures treated as above described were incubated for 72 h at 37 °C.

### Electrophoresis

After treatment as previously described, cells were washed twice in PBS, detached with ice-cold PBS, collected and centrifuged at 600 g for 10 min. The supernatant were then removed and the pellet were harvested and lysed by ice-cold lysis [1% (v/v) Triton X-100, 20 mM Tris-HCl, 137 mM NaCl, 10% (v/v) glycerol, 2 mM EDTA, 1mM phenylmethylsulfonyl fluoride (PMSF), 20 µM leupeptin hemisulfate salt, 0.2 U/mL aprotinin (all from Sigma Aldrich)] for 30 min on an icebath. Lysated were centrifuged at 13800 g for 20 min at 4°C. The protein concentration in the supernatant were spectrophotometrically determined by Bradford's protein assay [30], and the lysate were subjected to SDS-PAGE (NuPage Electrophoresis System- Invitrogen). Protein sample were diluted with NuPage LDS Sample Buffer 4x 1:4 (v/v) and NuPage Sample Reducing Agent with 1:10 (500mM dithiothreitol (DTT) at 10x concentration) then undergo to 70 °C for 10 min. Protein (30 µg lane-1) and prestained standards (Invitrogen) were loaded on 4-12% Novex Bis-Tris Midi gel 1.0mm precast gels (Life Technologies-Invitrogen).

### Western blotting

After electrophoresis, the resolved proteins were transferred from the gel to nitrocellulose membranes using iBlot Dry Blotting System A (Life-Tecnologies-Invitrogen). Then, membranes were blocked by PBS, (pH 7.2) contained 0.1% (v/v) Tween 20 and 5% (w/v) non-fat dried milk for 1h and washed three times with 0.1% Tween 20-PBS (T-PBS). Specific protein were detected using optimal concentration of rabbit anti-plkB-α ; mouse monoclonal anti-human P-glycoprotein (MDR); mouse monoclonal anti-TLR4, according to the Manufactured instructions (Santa Cruz Biotechnology; BD Bioscience).

Membranes were incubated with HRP-conjugated secondary antibodies (goat anti-rabbit IgG and goat anti-mouse IgG ; cat. A120-101 P cat. A90-116P 1:10000) (Bethyl laboratories) for 60 min at room temperature in the dark on a shaker. Finally, after three washings with T-PBS, bands were visualized by chemiluminescent method (Immun-Start™Western™ Kit-Biorad). The β-actin protein level was used as a protein loading control in western blotting.

### Treatment of cell cultures with small interfering RNA

Cell cultures (60-70% confluence) were submitted to TLR-4 or P-gp specific small interfering RNA (siRNA) using commercial kits (Santa Cruz Biotechnology) according to the manufacturer's protocol. Cells were incubated with 60-80 pmols of siRNA for 5-7 h at 37°C, using a transfection reagent. Then, cells were added with 1 mL of complete medium (2×) and incubated for 24 h. The day after, medium was replaced with fresh culture medium and cells were incubated for 48 h before experimental treatments. After 48 h of transfection, 30 mg of cell lysate was subjected to immunoblotting.

## Claims

1. A compound of the following formula I: 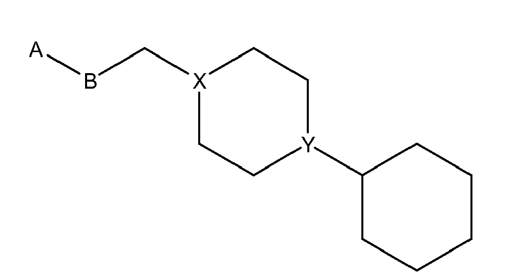 wherein :
- **B** is selected from 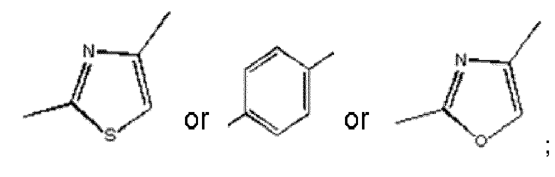 and
- **A** is selected from 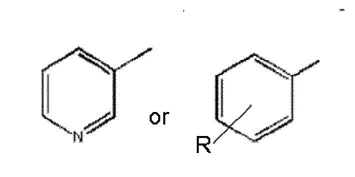
wherein **R** is: H, OH, OCH₃, Cl, Br, F; or NO₂; and
- **X** and **Y** are selected from CH or N.

2. The compound according to claim 1 wherein
**B** is

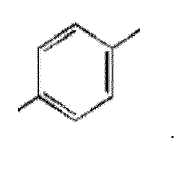

3. The compound according to claim 1 or 2 wherein
**A** is

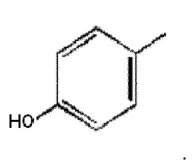

4. The compound according to any one of claims 1-3 wherein
**X** and/or **Y** are N.

5. The compound according to any one of claims 1-4, which is selected from
4'-(4-cyclohexyl-piperazin-1-yl-methyl)-biphenyl-4-R;
1-[2-(3-R)oxazol-4-yl-methyl]-4-cyclohexylpiperazine;
4'-(4-cyclohexyl-piperazin-1-yl-methyl)-biphenyl-2-R;
4'-(4-cyclohexyl-piperazin-1-yl-methyl)-biphenyl-3-R;
4'-(4-cyclohexyl-piperazin-1-yl-methyl)-phenyl-pyridine;
1-[2-(3-R-phenyl)oxazol-4-yl-methyl]-4-cyclohexylpiperazine;
1-[2-(2-R-phenyl)oxazol-4-yl-methyl]-4-cyclohexylpiperazine;
1-[2-(4-R-phenyl)oxazol-4-yl-methyl]-4-cyclohexylpiperazine;
1-[2-piridin-oxazol-4-yl-methyl]-4-cyclohexylpiperazine;
1-[2-(2-R-phenyl)tiazol-4-yl-methyl]-4-cyclohexylpiperazine;
1-[2-(3-R-phenyl)tiazol-4-yl-methyl]-4-cyclohexylpiperazine;
1-[2-(4-R-phenyl)tiazol-4-yl-methyl]-4-cyclohexylpiperazine;
1-[2-piridin-tiazol-4-yl-methyl]-4-cyclohexylpiperazine;
wherein in each compound R is selected from H, OH, OCH₃, Cl, Br, F; or NO₂.

6. The compound according to any one of claims 1-5, for use as a medicament

7. The compound according to claim 6, for use in the treatment and/or prevention of diseases **characterized by** a decrease in p-glycoprotein expression and/or a decrease in Toll like receptor 4 expression.

8. The compound according to claim 6 or 7, for use in the treatment and/or prevention of diseases **characterized by** an accumulation of beta-amyloid peptide.

9. The compound according to claim 8, wherein said diseases is selected from Alzheimer's disease and early stage of Alzheimer's disease.

10. A pharmaceutical composition comprising a compound according to any one of claims 1 to 5 and at least one pharmaceutical acceptable excipient.

11. The pharmaceutical composition according to claim 9, for use in the treatment and/or prevention of diseases **characterized by** an accumulation of beta-amyloid peptide.

12. The pharmaceutical composition according to claim 11, wherein said diseases is selected from Alzheimer's disease and early stage of Alzheimer's disease.

13. A method for synthesizing the compound according to claim 1, comprising the following step of: reducing the compound of formula (VI) to obtain the compound of formula (I), according to the following scheme (Scheme I)

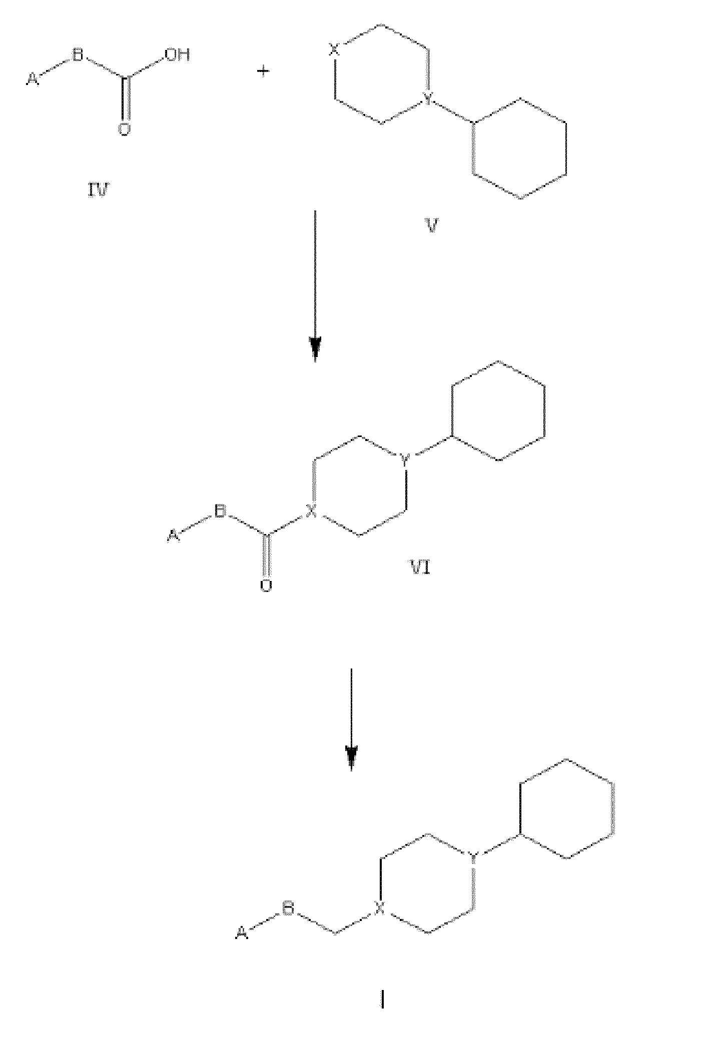

14. The method of claim 11, further comprising the step of reacting compound of formula (IV) with compound of formula (V) to generate the intermediate compound (VI).

15. A compound of the following formula (VI), as intermediate for the synthesis of the compound of formula (1):
